# EUROPEAN PATENT APPLICATION

(11) **EP 0 535 974 A1**
(43) Date of publication of application: **07.04.1993**
(21) Application number: 92309003.9
(22) Date of filing: 02.10.1992
(51) Int. Cl.: A61B 17/34

(54) **Flexible trocar tube**

(30) Priority: 04.10.1991 US 771489
(71) Applicant: ETHICON INC., Somerville New Jersey 08876 (US)
(72) Inventor: Pichon, Dean, Concord, Massachusetts 01742 (US); Lucas, Robert M., Lynnfield, Massachusetts 01940 (US)
(74) Representative: Fisher, Adrian John

(57) **Abstract**

A trocar assembly that includes a resiliently flexible trocar tube (12) having a relatively low bending stiffness and an inherent memory causing it to return to a generally straight orientation. The trocar tube is made from a flat-wire stainless steel reinforcing spring member (42) that is encased in an insulating elastomeric material.

## Description

### Field Of The Invention

This invention generally relates to surgical instruments. More particularly, the invention relates to a trocar assembly for providing communication into an anatomical cavity, that includes a flexible trocar tube.

### Background Of The Invention

A trocar assembly is a surgical instrument that is used to gain access to a body cavity. A trocar assembly generally comprises two major components, a trocar tube and an obturator. The trocar tube is inserted through the skin to access a body cavity through the tube in which laparoscopic or arthroscopic surgery and endoscopic procedures are to be performed. In order to penetrate the skin, the distal end of the trocar tube is placed against the skin and an obturator is inserted through the tube. The obturator has a sharp point or cutting edge at its distal end. By applying pressure against the proximal end of the obturator, the sharp point is forced through the skin until it enters the body cavity. The trocar tube is inserted through the perforation made by the obturator and the obturator is withdrawn, leaving the trocar tube as an access-way to the body cavity. Examples of trocar assemblies are disclosed in U.S. Patent No. 4,535,773.

Trocar tubes have heretofore generally been made from a rigid material. It is desirable to provide a trocar tube that permits curved, bent, flexible and/or articulating instruments to pass therethrough. It is also desirable that such a trocar tube be capable of bending at least 90 degrees and inherently returning to its straight position. It is further desirable that the trocar tube be electrically insulated to facilitate its use with electrical instruments such as cautery instruments.

There is a need for a trocar assembly having a resiliently flexible trocar tube that has an inherent memory causing it to return to a generally straight orientation. The flexible trocar tube should also be insulated and exhibit high hoop stiffness to resist collapse or distortion as external loads are applied.

### Summary Of The Invention

In accordance with the principles of the present invention, a trocar assembly is provided that includes a resiliently flexible trocar tube having a relatively low bending stiffness and an inherent memory causing it to return to a generally straight orientation. The trocar tube exhibits a high hoop stiffness to resist collapse or distortion of its cross section as external loads are applied.

In accordance with a preferred embodiment of the invention, the trocar tube is made from a flat-wire stainless steel reinforcing spring having closely spaced coils. The spring is encased in an insulating elastomeric material. In accordance with an alternative embodiment, the spring may be made from a non-metallic material. The cross section of the trocar tube may be round or oval.

### Brief Description Of The Drawings

A more complete appreciation of this invention, and many of the attendant advantages thereof, will be readily apparent as the same becomes better understood by reference to the following detailed description when considered in conjunction with the accompanying drawings in which like reference numerals indicate the same or similar components, wherein:
FIG. 1 is a cross-sectional view of an exemplary trocar assembly that includes a flexible trocar tube constructed in accordance with the present invention; and
FIG. 2 is an enlarged cross-sectional view of a portion of the trocar tube shown in FIG. 1.

### Detailed Description Of The Preferred Embodiments

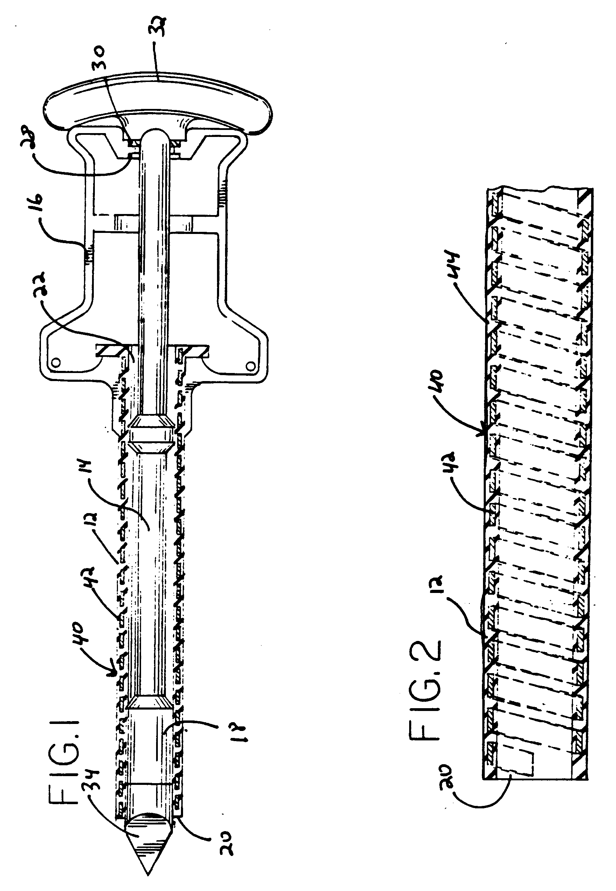

Referring to FIG. 1, there is shown a trocar assembly 10 that includes a flexible trocar tube 12 constructed in accordance with the invention. The particular construction of the trocar assembly does not form a part of the invention and the disclosure thereof is merely for exemplary purposes. Alternative trocar assemblies that may incorporate a flexible trocar tube 12 are disclosed in EP-A-474124.

Trocar assembly 10 includes a trocar tube 12, an obturator 14, and a housing or handle 16. Trocar tube 12 defines an interior lumen 18 having an open distal end 20 and an open, flanged proximal end 22. The proximal end 22 extends into and is mounted in handle 16. The proximal end of the handle is formed with an aperture 28 that is surrounded by a sealing gasket ring 30 of known configuration.

An obturator 14 is slideably and removably extendable within trocar tube 12 and is inserted into handle 16 and trocar tube 12 through aperture 28. An obturator handle 32 is provided at the proximal end of the obturator and a sharpened point or blade 34 is formed at the distal end thereof.

As is well known in the art, trocar assembly 10 is used to puncture a hole in soft tissue by positioning the point 34 of the obturator 14 against the tissue, and pressing against the obturator handle 32. The obturator point 34 breaks through the inner surface of the tissue and the trocar tube 12 is directed into the body cavity. After the puncture is made, the obturator 14 is withdrawn from the trocar tube 12 by handle 32.

Referring to FIG. 2, there is shown a portion of a flexible trocar tube 12 constructed in accordance with a preferred embodiment of the invention. Trocar tube 12 is formed from a sleeve 40 made of a flat-wire stainless steel reinforcing spring having closely spaced coils 42. The reinforcing spring is encased in a sheath 44 made of an insulating elastomeric material, such as polyurethane or the like. The inner and outer surfaces of coils 42 are preferably encased in sheath 44.

The selection of the particular material and construction of the reinforcing sleeve 40 and the material of the sheath 44 is such that trocar tube 12 has sufficient flexibility to permit it to bend at least about 90 degrees while substantially maintaining its cross sectional shape in the bent portion of the tube. The resiliency of tube 12 is such that it has an inherent memory causing it to return to a generally straight orientation upon removal of the bending force applied thereto. The bending stiffness of tube 12 is preferably relatively low so that a low bending force is required to cause tube 12 to achieve a curved or bent orientation. It is highly desirable that tube 12 have a sufficiently high hoop stiffness to resist collapse or distortion of its cross section as expected external loads are applied. In accordance with a preferred embodiment, the effective Young's Modulus of tube 12 is at least about 800,000 psi when measured in the hoop direction.

There are many design variables that contribute to the resiliency, bending stiffness and hoop stiffness properties of tube 12. Among such variables are the particular material, the shape, the thickness and the pitch of the wire coil 42. Additionally, the properties of tube 12 are effected by the particular material of sheath 44 and the durometer thereof.

The flexible sleeve member 40 may be made from a flat-wire reinforcing spring made from a metallic material such as stainless steel. Alternatively, sleeve member 40 may be made from a suitable thermoplastic material that is invisible to X-rays. The cross section of the coils 42 may be either generally flat or round. The cross section of sleeve 40 may be round or oval.

In use, trocar tube 12 is directed into the patient's body cavity in a straight orientation in a well known manner as discussed above. Insertion of a fixed curved shaft or articulated instruments into tube 12 causes the tube to bend as necessary to accommodate the passage of the instrument therethrough. The encased inner surface of tube 12 facilitates the passage and the manipulation of the instrument. Upon removal of the instrument from tube 12, it returns to its generally straight orientation ready for receipt of another instrument or for removal from the patient.

From the foregoing, it will be observed that numerous modifications and corrections can be effected without departing from the true spirit and scope of the novel concepts of the present invention. It will be understood that no limitation with respect to the specific embodiment illustrated herein is intended or should be inferred. It is, of course, intended to cover by the appended claims all such modifications as fall within the scope of the claims.

## Claims

1. A trocar assembly for providing communication into an anatomical cavity, comprising:
a trocar tube defining an interior lumen having an open proximal end portion and an open distal end portion for accommodating axial reciprocation of an elongate implement of lesser cross-sectional dimension therethrough, said trocar tube being formed of a resiliently flexible sleeve member having a relatively low bending stiffness and an inherent memory permitting it to bend upon application of a bending force and causing it to return to a generally straight orientation upon removal of the bending force.

2. The trocar assembly as defined in claim 1 wherein said trocar tube has an effective Young's Modulus of at least about 800,000 psi when measured in the hoop direction to provide a means for maintaining the cross sectional shape of said sleeve member.

3. The trocar assembly as defined in claim 1 wherein said flexible sleeve member is a reinforcing spring member having closely spaced coils.

4. The trocar assembly as defined in claim 3 wherein said reinforcing spring member is made from generally flat wire.

5. The trocar assembly as defined in claim 3 wherein said reinforcing spring member is made from generally round wire.

6. The trocar assembly as defined in claim 3 wherein said reinforcing spring member is a metallic material.

7. The trocar assembly as defined in claim 3 wherein said reinforcing spring member is stainless steel.

8. The trocar assembly as defined in claim 3 wherein said reinforcing spring member is a non-metallic material.

9. The trocar assembly as defined in claim 3 wherein said reinforcing spring member is encased in an elastomeric material.

10. The trocar assembly as defined in claim 9 wherein said elastomeric material covers the inner surface and the outer surface of said reinforcing spring member.

11. The trocar assembly as defined in claim 10 wherein said elastomeric material extends between said coils.

12. The trocar assembly as defined in claim 9 wherein said elastomeric material is polyurethane.

13. The trocar assembly as defined in claim 1 wherein the bending stiffness said trocar tube cooperate to permit said trocar tube to bend at least about 90 degrees.
